# EUROPEAN PATENT APPLICATION

(11) **EP 1 767 620 A1**
(43) Date of publication of application: **28.03.2007**
(21) Application number: 05726653.8
(22) Date of filing: 17.03.2005
(51) Int. Cl.: C12N 5/10, C12Q 1/68

(54) **EXHAUSTIVE GENE EXPRESSION PROFILING ANALYSIS USING MICROSAMPLE**

(30) Priority: 03.06.2004 JP 2004165208
(71) Applicant: National Institute of Radiological Sciences, Chiba-shi, Chiba-ken 263-8555 (JP)
(72) Inventor: ABE, Masumi c/o NAT. INST. OF RADIOLOGICAL SC., Chiba-shi, Chiba 2638555 (JP); ARAKI, Ryoko c/o NAT. INST. OF RADIOLOGICAL SC., Chiba-shi, Chiba 2638555 (JP)
(74) Representative: Strehl Schübel-Hopf & Partner
(86) International application number: PCT/JP2005/004788
(87) International publication number: WO 2005/118791

(57) **Abstract**

The purpose of the present invention is to prepare a cellular gene expression profile from an extremely small number of cells so as to realize its application to pathological samples, microtisuues, microanimals, etc, whose handling has been infeasible because of a limited sample amount, and further to realize a gene expression profile as to cells of peripheral blood for use in pathological diagnosis, etc. There is provided a method attaining improvement of the high coverage expression profiling analysis (HiCEP) disclosed in the pamphlet of WO 02/48352, **characterized in that** an amount of mRNA contained in a starting material is increased through obtaining amplified RNA complementary to double-stranded cDNA sequence by the action of RNA polymerase, and that the number of double-stranded cDNAs having X primer and Y primer added thereto is increased by the use of PCR.

## Description

### Field of the invention

The invention relates to a method that has improved High Coverage Expression Profiling (HiCEP) analysis disclosed in WO02/48352 pamphlet, which enables the preparation of a satisfying gene expression profile even from an extremely small amount of samples.

### Background of the invention

The human genome sequence was almost completely determined in 2002. It is expected to develop made-to-order therapeutic drugs on the basis of the thus determined base sequences. For that purpose, it is very important to reveal which gene and how much is expressed in human body, that is, network of gene expression. And it will be necessary to prepare gene expression profile showing which gene and how much is expressed at a particular point of time in human body for revealing the network of gene expression.

The currently used methods for the preparation of the gene expression profile include differential display method, sequential analysis of gene expression (SAGE), methods using micro array or DNA chip, and the above HiCEP. Among them, HiCEP is an excellent method as it can easily prepare the gene expression profile that may cover a wide variety of genes including unknown ones.

However, in any of the above-mentioned methods, it will be necessary to obtain as material a large amount of RNA, gene transcription product in order to prepare the gene expression profile with a high accuracy. Although a relatively small amount of RNA may serve well in the HiCEP, for example, it will still need to use from mRNA of 1.5 µg (about 75 µg in total RNA conversion according to Non-Patent Document 1) to 0.1 µg (about 5 µg in total RNA conversion). Usually, mRNA will be obtained in an amount of about 0.2 µg to 0.1 µg (about from 10 µg to 5 µg in total RNA conversion) from 10⁶ mouse culture cells under the best extracting conditions. This means that if living cells or tissues are used as material for study, they will be needed in the size of the little finger's tip, making almost impossible to actually obtain them in a clinical site, being in turn forced to obtain the gene expression profile at the sacrifice of accuracy.

On the other hand, T7 RNA amplification method (Non-Patent Document 2) is known for amplifying mRNA itself in case a large amount of material cannot be supplied. A general amplification ratio is 10 to 100 times in this method, and a required amount of the total RNA will be 0.1 to 0.01 µg, i.e., 10⁴ ~ 10³ cells. However, this method comprises many manipulation steps and is very complicate, causing the risk of increasing the degree of fluctuation per experiment.
[Patent Document 1] WO02/48352 pamphlet
[Non Patent Document 1] R, Fukumura, et al.: Nucleic Acids Research, 2003, Vol.31, No.16e94
[Non Patent Document 2] Eberwine, J. et al.:Proc. Natl. Acad. Sci. USA, 89:3010-3014, 1992

### Disclosure of the Invention

### Problems to be solved by the Invention

The expression amount of each gene is different depending on its kind and timing of expression. It is therefore desired that a method for preparing the gene expression profile should have a high detection sensitivity in order to reveal the gene expression network.

The purpose of the present invention is therefore to provide a method for preparing gene expression profile from cells with a number of less than 10³, realizing its application to pathological samples, microtissues, microanimals, etc. whose handling has been infeasible because of a limited sample amount and peripheral blood cells for use in pathological diagnosis.

### Means for solving the problems

A first aspect of the present invention relates to the following method.
A method for the preparation of gene expression profile comprising:
(a) a step of synthesizing a single-stranded cDNA whose 5' end is fixed to solid phase or which has a tag substance added to its 5' end with poly(A) RNA as a template;
(b) a step of synthesizing a double-stranded cDNA with the single-stranded cDNA synthesized in the step (a) as a template;
(c) a step of cleaving the double-stranded cDNA prepared in the step (b) with a first restriction enzyme X ;
(d) a step of purifying a 3' end fragment that is fixed to the solid phase or has the tag substance added thereto from the double-stranded cDNA fragments prepared in the step (c);
(e) a step of preparing a double-stranded cDNA fragment having an X promoter-adaptor bound to its 5' end by binding the X promoter-adaptor to a cleavage site with the first restriction enzyme X in the fragment purified in the step (d), wherein the X promoter-adaptor comprises a sequence complementary to the cleavage site, an X primer sequence and a promoter sequence;
(f) a step of preparing an amplified RNA (aRNA) complementary to the double-stranded cDNA sequence prepared in the step (e) with the double-stranded cDNA fragment as a template by means of an RNA polymerase recognizing the promoter sequence;
(g) a step of synthesizing a single-stranded cDNA having a sequence complementary to the X primer with the aRNA synthesized in the step (f) as a tempelate;
(h) a step of synthesizing a double-stranded cDNA whose 5' or 3' end is fixed to solid phase or which has tag substance added to its 5' or 3' end with the single-stranded cDNA synthesized in the step (g) as a template;
(i) a step of cleaving the double-stranded cDNA synthesized in the step (h) with a second restriction enzyme Y that does not cleave the X primer sequence at its 5' end;
(j) A step of purifying a double-stranded cDNA fragment comprising a cleavage site with the second restriction enzyme Y at its 3' end from the fragments prepared in the step (i);
(k) a step of preparing a double-stranded cDNA fragment having a Y adaptor bound to its 3' end by binding the X promoter-adaptor to a cleavage site with the second restriction enzyme Y in the double-stranded cDNA fragment purified in the step (j), wherein the Y adaptor comprises a sequence complementary to the cleavage site and a Y primer;
(l) a step of amplifying the double-stranded cDNA fragment prepared in the step (k) by means of PCR with the use of said double-stranded cDNA fragment as a template and a primer set of the X primer and Y primer;
(m) a step of PCR with the use of the double-stranded cDNA sequence prepared in the step (1) as a template and a primer set of X1 primer comprising two-base sequence of N₁N₂ (N₁ and N₂ are a base selected from the group consisting of adenine, thymine, guanine and cytosine, being the same or different with each other) at its 3' end and Y1 primer comprising two base sequence of N₃N₄ (N₃ and N₄ are a base selected from the group consisting of adenine, thymine, guanine and cytosine, being the same or different with each other) at its 3' end; and
(n) a step of subjecting the PCR product prepared in the step (m) to electrophoresis, and detecting migration length and peak so as to prepare the gene expression profile.

The present invention is characterized in that the amount of RNA comprised in a starting substance is increased by preparing an amplified RNA (aRNA) complementary to the double-stranded cDNA sequence by means of an RNA polymerase in the step (f), and that the number of the double-stranded cDNA having the X primer and Y primer added thereto is increased in the step (1) .

The step (1) may be omitted in the case where a sufficient amount of aRNA for the analysis of gene expression profile can be obtained in the step (f) in the first aspect of the present invention. A second aspect of the present invention therefore relates to the following method.

A method for the preparation of gene expression profile comprising:
(a) a step of synthesizing a single-stranded cDNA whose 5' end is fixed to solid phase or which has a tag substance added to its 5' end with poly(A) RNA as a template;
(b) a step of synthesizing a double-stranded cDNA with the single-stranded cDNA synthesized in the step (a) as a template;
(c) a step of cleaving the double-stranded cDNA prepared in the step (b) with a first restriction enzyme X ;
(d) a step of purifying a 3' end fragment that is fixed to the solid phase or has the tag substance thereto from the double-stranded cDNA fragments prepared in the step (c);
(e) a step of preparing a double-stranded cDNA fragment having an X promoter-adaptor bound to its 5' end by binding the X promoter-adaptor to a cleavage site with the first restriction enzyme X in the fragment purified in the step (d), wherein the X promoter-adaptor comprises a sequence complementary to the cleavage site, an X primer sequence and a promoter sequence;
(f) a step of preparing an amplified RNA (aRNA) complementary to the double-stranded cDNA sequence prepared in the step (e) with the double-stranded cDNA fragment as a template by means of an RNA polymerase recognizing the promoter sequence;
(g) a step of synthesizing a single-stranded cDNA having a sequence complementary to the X primer with the aRNA synthesized in the step (f) as a template;
(h) a step of synthesizing a double-stranded cDNA whose 5' or 3' end is fixed to solid phase or which has tag substance added to its 5' or 3' end with the single-stranded cDNA synthesized in the step (g) as a template;
(i) a step of cleaving the double-stranded cDNA synthesized in the step (h) with a second restriction enzyme Y that does not cleave the X primer sequence at its 5' end;
(j) A step of purifying a double-stranded cDNA fragment comprising a cleavage site with the second restriction enzyme Y at its 3' end from the fragments prepared in the step (i);
(k) a step of preparing a double-stranded cDNA fragment having a Y adaptor bound to its 3' end by binding the X promoter-adaptor to a cleavage site with the second restriction enzyme Y in the double-stranded cDNA fragment purified in the step (j), wherein the Y adaptor comprises a sequence complementary to the cleavage site and a Y primer;
(m) a step of PCR with the use of the double-stranded cDNA sequence prepared in the step (1) as a template and a primer set of X1 primer comprising two-base sequence of N₁N₂ (N₁ and N₂ are a base selected from the group consisting of adenine, thymine, guanine and cytosine, being the same or different with each other) at its 3' end and Y1 primer comprising two base sequence of N₃N₄ (N₃ and N₄ are a base selected from the group consisting of adenine, thymine, guanine and cytosine, being the same or different with each other) at its 3' end; and
(n) a step of subjecting the PCR product prepared in the step (m) to electrophoresis, and detecting migration length and peak so as to prepare the gene expression profile.

The steps (f) ~ (h) may be further omitted in the case where a sufficient amount of the double-stranded cDNA for the analysis of gene expression profile can be amplified in PCR if the step (1) in the first aspect of the present invention. A third aspect of the present invention therefore relates to the following method.

A method for the preparation of gene expression profile comprising:
(a) a step of synthesizing a single-stranded cDNA whose 5' end is fixed to solid phase, or which has tag substance added to its 5' end with poly(A) RNA as a template;
(b) a step of synthesizing a double-stranded cDNA with the single-stranded cDNA synthesized in the step (a) as a template;
(c) a step of cleaving the double-stranded cDNA prepared in the step (b) with a first restriction enzyme X;
(d) a step of purifying a 3' end fragment that is fixed to the solid phase or has the tag substance added thereto from the double-stranded cDNA fragments prepared in the step (c);
(e) a step of preparing a double-stranded cDNA fragment having an X promoter-adaptor bound to its 5' end by binding the X promoter-adaptor to a cleavage site with the first restriction enzyme X in the fragment purified in the step (d), wherein the X promoter-adaptor comprises a sequence complementary to the cleavage site, an X primer sequence and a promoter sequence;
(i) a step of cleaving the double-stranded cDNA synthesized in the step (e) with a second restriction enzyme Y that does not cleave the X primer sequence at its 5' end;
(j) a step of purifying a double-stranded cDNA fragment comprising a cleavage site with the second restriction enzyme Y at its 3' end from the fragments prepared in the step (i);
(k) a step of preparing a double-stranded cDNA fragment having a Y adaptor bound to its 3' end by binding the X promoter-adaptor to a cleavage site with the second restriction enzyme Y in the double-stranded cDNA fragment purified in the step (j), wherein the Y adaptor comprises a sequence complementary to the cleavage site and a Y primer;
(l) a step of amplifying the double-stranded cDNA fragment prepared in the step (k) by means of PCR with the use of said double-stranded cDNA fragment as a template and a primer set of the X primer and Y primer;
(m) a step of PCR with the use of the double-stranded cDNA sequence prepared in the step (1) as a template and a primer set of X1 primer comprising two-base sequence of N₁N₂ (N₁ and N₂ are a base selected from the group consisting of adenine, thymine, guanine and cytosine, being the same or different with each other) at its 3' end and Y1 primer comprising two base sequence of N₃N₄ (N₃ and N₄ are a base selected from the group consisting of adenine, thymine, guanine and cytosine, being the same or different with each other) at its 3' end; and
(n) a step of subjecting the PCR product prepared in the step (m) to electrophoresis, and detecting migration length and peak so as to prepare the gene expression profile.

### Advantages of the Invention

According to the present invention, the amount of mRNA originally comprised in the starting substance can be finally increased by about 10,00 ~ 500,000 times by being increased, for example, by about 10 to 500 times in the step (f), for example, by 128 to 1,024 times in the step (1) . As a result, the number of cells conventionally used in the HiCEP may be reduced from 10⁶ to 10³ ~ one or a few (from about 10 ng to about 20 pg in total RNA conversion). These reduced numbers of the cells are less than 10³ that has been considered as a lower limit in DNA chip, realizing its application to pathological samples, microtissues, microanimals, etc. whose handling has been infeasible because of a limited sample amount.

### Brief description of Drawings

Fig. 1 shows an outline of the step (a) of the present invention.
Fig. 2 shows an outline of the steps (b) and (c) of the present invention.
Fig. 3 shows an outline of the steps (d) and (e) of the present invention.
Fig. 4 shows an outline of the step (f) of the present invention.
Fig. 5 shows an outline of the step (g) of the present invention.
Fig. 6 shows an outline of the step (h) of the present invention.
Fig. 7 shows an outline of the steps (i) and (j) of the present invention.
Fig. 8 shows an outline of the step (k) of the present invention.
Fig. 9 shows an outline of the step (1) of the present invention.
Fig. 10 shows an outline of the step (m) of the present invention.
Fig. 11 shows an outline of the step (n) of the present invention.
Fig. 12 shows the results of gene expression profile analysis using total RNA 10 µg (10⁶ cells) (T10uLot1 with a black line, and T10uLot2 with a blue line).
Fig. 13 shows the results of gene expression profile analysis using total RNA 10 ng (10³ cells) (T10nLot1 with a black line, and T10nLot2 with a blue line).
Fig. 14 shows the results of gene expression profile analysis using total RNA 10 pg (two cells) (T10pLot1 with a black line, and T10pLot2 with a blue line).

### Best mode for carrying out the invention

The present invention is characterized mainly by improving the cDNA preparing step and the PCR amplifying step in the HiCEP method.

The HiCEP method is a gene expression profiling method that was developed on the basis of Restriction enzyme DNA Fragment Length Polymorphism (PFLP) and Polymerase Chain Reaction (PCR) . According to this method, gene expression profile is obtained based on the data about migration length and peak in electrophoresis of the PCR product. The gene expression profile comprises information about gene expression patterns, the presence or absence of known and unknown genes, and their expression amount, etc of a particular-type cell under particular conditions. By using the profile thus obtained, frequency of gene expression can be analyzed and each gene can be identified. In the HiCEP method, a false positive signal can be reduced to 2% or less, attaining such a very high coverage ratio as 80% (the ratio of observable transcripts for the total transcripts) and enabling the detection of expression difference of even as small as 1.2 times.

It is preferable to carry out annealing of the X primer or X1 primer, and the Y primer oy Y1 primer with the X adaptor and Y adaptor, respectively, in the step (1) and/or (m) at a temperature range of TmMAX + 6°C ~ TmMAX + 14°C of the primer, so that the occurrence of false peaks due to mis-annealing of the primers can be diminished. Unless otherwise noted in the specification, 5' end of a sense chain (a chain homologous to a poly (A) RNA serving as a template) of a double-stranded cDNA means 5' end of the double-stranded cDNA, and 3' end of the sense chain means 3' end of the double-stranded cDNA.

The "restriction enzyme" is an enzyme also called a "restriction endonuclease", which will hydrolyze and cleave the double-stranded DNA at a particular sequence. Two kinds of the restriction enzymes "X" and "Y" are used in combination according to the present invention in order to obtain an appropriate fragment. It is preferable to use restriction enzymes in the present invention, which are able to cleave the double-stranded cDNA synthesized from mRNA of the expressed gene into fragments with an identifiable length. Further, it is preferable to use enzymes that can cleave as many double-stranded chains as possible, preferably almost all of them. For example, 6 or 4 base-recognizing enzymes known for those skilled in the art such as those described in WO02/48352 pamphlet may be used. As already described above, it is preferable to use the 4 base-recognizing enzymes such as MspI and MseI together in order to attain the high coverage ratio.

As the adaptor comprises a sequence complementary to the cleavage site of the first or second restriction enzyme, it can bind to the cleavage site. It also comprises the X primer sequence or Y primer sequence, so that a sequence located between these primers may be amplified in the step (1) by PCR using these primers. It may be optionally designed depending on the structure of the restriction enzymes and primers used in the reaction. The primers are usually 30-base long for performing a stable PCR.

The X primer, X1 primer, Y primer and Y1 primer have preferably 16 bases or more so as not to coincide with the subject RNA sequence wherever possible. Furthermore, it is necessary for these primers to satisfy the conditions generally required as a PCR primer, such as those described in "BioRad Experiment Illustrated (3) New Edition, Really Amplified PCR" Hiroki Nakayama, Shujunn Co., 2002, the second edition, the forth print. Each primer may be prepared in accordance with a general synthesizing method known for those skilled in the art (Letsinger et al., Nucleic Acids Research, 20, 1879-1882, 1992; Japanese Patent Application Publication Hei.11(1999)-08018).

It is further preferable to bind a labeling substance such as any fluorescent substance known for those skilled in the art to at least either end of the primers in order to ease the detection after PCR. For example, the suitable fluorescent substances include 6-carboxyfluorescein (FAM), 4,7,2',4',5',7'-hexachloro-6-carboxyfluorescein (HEX), NED (Applied System Japan, Co.) and 6-carboxy-X-rhodamine (Rox).

The degree of the amplification in the steps (f) and/or (1) of the present invention may be optionally determined by those skilled in the art depending on the starting substance, subject substance (the amount of mRNA originally comprised therein), the kind of polymerase and promoter sequence, and reaction conditions in each step. It is, however, necessary to maintain a ratio among the amounts of each mRNA originally comprised in the subject substance during the amplification steps (s) in order to accurately analyze the gene expression profile. For that purpose, it is preferable to obtain the amplified RNA in an amount of about 10 to 500 times as much as the number of the double-stranded cDNA fragments in the step (f). And, it is preferable to amplify the number of the double-stranded cDNA fragment by 128 to 1, 024 times by repeating PCR in 7 to 10 cycles in the step (1).

There is no particular limitation on the RNA polymerase and promoter sequence used in the present invention and any ones known for those skilled in the art may be used. For example, there may be mentioned T3 or T7 promoter sequences derived from phage that infects E. coli, and SP6 promoter sequence, and RNA polymerases that can bind to these sequences.

When the double-stranded cDNA whose 5' end is fixed to solid phase or which has tag substance added to its 5' end is synthesized in the step (h) with the single-stranded cDNA synthesized in the step (g) as a template, an oligomer may be used as a primer for a complementarily synthesized cDNA, which comprises the X primer sequence or a part thereof fixed to the solid phase or having the tag substance added thereto. Alternatively, when the double-stranded cDNA whose 3' end is fixed to solid phase or which has tag substance added to its 3' end is synthesized in the step (h) with the single-stranded cDNA synthesized in the step (g) as a template, an oligo T primer fixed to the solid phase such as oligo T beads or having the tag substance added thereto may be used in the step (g) like in the step (a).

Furthermore, depending on the fact that the double-stranded cDNA fragment prepared in the step (h) is fixed via either 5' end or 3' end, or has the tag substance added to either its 5' end or 3' end, the double-stranded cDNA fragment fixed to the solid phase or having the tag substance shall be subjected to purification/collection or removal.

The solid phase may be optionally selected from any substances known for those skilled in the art, such as polystyrene beads, magnetic beads and silica-gel beads.

The tag substance and a substance having a high affinity for the tag substance mean one of the substances that can specifically bind with each other with a high affinity. Any substances may be used for them as long as they specifically bind with each other with a high affinity. Unlimited examples of the combination of these substances include biotin and streptavidin, biotin and avidin, FITC and anti-FITC antibody, DIG and anti-DIG, protein A and mouse IgG, and latex particles, etc. The tag substance may be added to the DNA sequence under any suitable conditions known for those skilled in the art. Eacheach sequence may be further fixed to the solid phase through the reaction between the tag substance and the substance having a high affinity for the tag substance.

The double-stranded cDNA fragment having the tag substance added thereto may be collected by means of a specific reaction between the tag substance and the substance having a high affinity for the tag substance. The double-stranded cDNA fragment fixed to the solid phase may be easily collected by removing other fragments from a reaction system with washing. These reactions may be carried out under any suitable conditions known for those skilled in the art.

Other conditions and apparatuses used in the HiCEP method may be referred to the description of WO02/48352 pamphlet. The resulting gene expression profile may be analyzed by means of any analyzing software known for those skilled in the art such as GeneScan (a trade mark: Applied BioSystems Japan, Co.)

### Examples

Next, the invention will be described in detail with reference to an example corresponding to the first aspect of the present invention, however, it should be appreciated that the invention will not be limited in any way by the specific example. An outline of the example is shown in Fig.1 - Fig.11

### Material

TT2 cells (Invitrogen Co.) were cultured to obtain 10⁷ cells. Using the thus obtained cells, 100 µg of total RNA was obtained by means of RNAeasy Total RNA extraction kit (Invitrogen Co.). The resulting total RNA was divided into each volume shown in the following Sample Table and used as test material. In order to secure reproducibility of the experiments, each sample was prepared into two Lots.

**[Table 1]**

| Sample Table | | | |
|---|---|---|---|
| Sample Name | Total RNA amount | Used amount (Concentration) | Number of cells (Conversion) |
| T10u | 10µg | 1 µl (10µg/µl) | 10⁶ |
| T10n | 10ng | 1 µl (10ng/ µl) | 10³ |
| T20p | 20pg | 1 µl (20pg/µl) | 2 |

(a) Step of synthesizing a single-stranded cDNA whose 5' end is fixed to solid phase with poly(A) RNA as a template:

Synthesized dT30V oligomer modified with Biotin at its 5' (SEQ ID No. 1) (2.6µg : about 250 pmol) : Biotin-TTTTTTTTTTTTTTTTTTTTTTTTTTTTTTV (V = A or C or G) and 1ml of Dynabeads M280 (Streptavidin-coated magnetic beads: DYNAL Co.) were mixed at a room temperature for 30 min. to give rise to Biotin-Streptavidin reaction. After the resulting solution of the dT30V oligomer fixed on the magnetic beads was placed and adsorbed onto magnet, supernatant was removed so as to exclude un-reacted dT30V oligomer. The collected dT30V oligomer fixed on the magnetic beads was dissolved in 10µl of water to obtain the dT30V oligomer fixed on the magnetic beads. Each 1µl of the solution of the dT30V oligomer fixed on the magnetic beads thus obtained was added into T10uLot1 solution (1µl : conc. 10µg/µl), T10uLot2 solution (1µl : conc. 10µg/µl), T10nLot1 solution (1µl : conc. 10ng/µl), T10nLot2 solution (1µl : conc. 10ng/µl), T20pLot1 solution (1µl : conc. 20pg/µl), and T20pLot2 solution (1µl : conc. 20pg/µl), respectively. As the following procedures were done in the same way for all of the samples and lots, T10uLot1 will hereinafter be used as their representative. 1^{st} cDNA synthesis using T10uLot1 in accordance with SuperScript III Firs-Strand Synthesis System (Invitrogen Co.) and its protocol.

**[Table 2]**

| Primer annealing solution | |
|---|---|
| Solution of dT30V oligomer fixed on the magnetic beads | |
| | 1µl |
| T10uLot1 | 1µl |
| 10mM dNTPMix | 0.5µl |
| Total | 2.5µl |

The above solution was incubated at 65°C for 5 min to carry out hybridization between mRNA and the dT30V oligomer fixed on the magnetic beads. The following solution for the 1^{st} cDNA synthesis was then added thereto.

**[Table 3]**

| Solution for the 1^{st} cDNA synthesis | |
|---|---|
| 10X RT buffer | 0.5µl |
| 25mM MgCl₂ | 1µl |
| 0.1M DTT | 0.5µl |
| Rnase Out (40u/µl) | 0.25µl |
| SuperScript III RT (200u/µl) | 0.25µl |
| Total | 2.5µl |

The resulting solution was allowed to react at 50°C for 60 min, and then heated at 85°C for 5 min to inactivate SuperScript III RT.

(b) Step of synthesizing a double-stranded cDNA with the single-stranded cDNA synthesized in the step (a) as a template:

**[Table 4]**

| Solution for the 2nd cDNA synthesis | |
|---|---|
| Solution of the step (a) | 5µl |
| Water | 21.25µl |
| 5X 2^{nd} Strand buffer | 7.5µl |
| 10mM dNTPMix | 1µl |
| 0.1M DTT | 1µl |
| E. coli DNA ligase (1u/µl) | 0.5µl |
| E. coli DNA polymerase (10u/µl) | 1µl |
| E. coli RNAse H (1u/µl) | 0.25µl |
| Total | 37.5µl |

The above reaction solution was allowed to stand at 16°C for 120 min and incubated at 70°C for 15 min. The resulting solution was placed on magnet so that the magnetic beads would be adsorbed onto the magnet. Supernatant was then removed so as to exclude unwanted enzymes and un-reacted agents. The magnetic beads were washed two times with 500µl of 0.1X TE buffer. Finally, after the addition of water (40.4µl), the magnetic beads were detached from the magnet and dispersed in the water.

(c) Step of cleaving the double-stranded cDNA prepared in the step (b) with a first restriction enzyme X :

**[Table 5]**

| Solution for cleavage with restriction enzyme MspI | |
|---|---|
| Solution of the step (b) | 40.4µl |
| 10X T buffer | 5.3µl |
| 0.1 BSA | 5.3µl |
| Msp I (3u/µl) | 2µl |
| Total | 53µl |

The above solution was allowed to react at 37°C for 4 hours.

(d) Step of purifying a 3' end fragment that is fixed to the solid phase from the double-stranded cDNA fragments prepared in the step (c):
The reaction solution obtained in the step (c) was placed on magnet so that the magnetic beads would be adsorbed onto magnet. Supernatant was then removed so as to exclude unwanted enzymes and un-reacted agents. The magnetic beads were washed two times with 500µl of 0.1X TE buffer. Finally, after the addition of water (20µl), the magnetic beads were detached from the magnet and dispersed in the water.

(e) Step of preparing a double-stranded cDNA fragment having an X promoter-adaptor bound to its 5' end by binding the X promoter-adaptor to a cleavage site with the first restriction enzyme X in the fragment purified in the step (d), wherein the X promoter-adaptor comprises a sequence complementary to the cleavage site, an X primer sequence and T7 promoter sequence:

Preparation of X-T7 adaptor
Synthesized DNA oligomer:
X-T7S (SEQ ID No.2)
TAGGTAATACGACTCACTATAGGGAATGGCTACACGAACTCGGTTCATGACA
X-T7AS (SEQ ID No.3)
CGTGTCATGAACCGAGTTCGTGTAGCCATTCCCTATAGTGAGTCGTATTACCTA.
50µl of each DNA oligomer (100pmol/µl) was mixed together, heated at 95°C for 5 min, and allowed to cool down to a room temperature so as to prepare solution of X-T7 adaptor of double-stranded DNA (50pmol/µl).

**[Table 6]**

| Ligation of X-T7 adaptor | |
|---|---|
| Solution of the step (d) | 20µl |
| X-T7 adaptor solution (50pmol/µl) | 2µl |
| 10X T4 DNA ligase buffer | 2.8µl |
| 10mM ATP | 1µl |
| T4 DNA ligase | 2µl |
| Total | 27.8µl |

The above solution was heated at 25°C for 4 hours and placed on magnet so that the magnetic beads would be adsorbed onto magnet. Supernatant was then removed so as to exclude unwanted enzymes and un-reacted agents. The magnetic beads were washed two times with 500µl of 0.1X TE buffer. Finally, after the addition of water (12µl), the magnetic beads were detached from the magnet and dispersed in the water.

(f) Step of preparing an amplified RNA (aRNA) complementary to the double-stranded cDNA sequence prepared in the step (e) in an amount of 10 to 500 times as much as that of the double-stranded cDNA using the double-stranded cDNA fragment as a template by means of T7 RNA polymerase:

**[Table 7]**

| Solution for synthesis of aRNA | |
|---|---|
| Water | 8µl |
| 5X RNA Polymerase buffer | 4µl |
| 250mM NaCl | 2µl |
| 10mM rATP | 1µl |
| 10mM rCTP | 1µl |
| 10mM rGTP | 1µl |
| 10mM rUTP | 1µl |
| Rnase Inhibitor (20-40u/µl) | 1µl |
| T7 RNA Polymerase (10u//µl) | 1µl |
| Total | 20µl |

The magnetic beads prepared in the step (e) were added to the solution for synthesis of aRNA (20µl) and suspended therein. The resulting suspension was heated at 40°C for 60 min, and then at 85 °C for 5 min. It was then placed and adsorbed onto the magnet so as to collect supernatant comprising the amplified RNA.

(g) Step of synthesizing a single-stranded cDNA having a sequence complementary to the X primer with the aRNA synthesized in the step (f) as a template:

Solution (50pM) of dT30V oigomer (SEQ ID No.1) was prepared. TTTTTTTTTTTTTTTTTTTTTTTTTTTTTTV (V = A or C or G)

**[Table 8]**

| Primer annealing solution | |
|---|---|
| Solution of aRNA prepared in the step (f) | 20µl |
| dT30V oligomer solution | 4µl |
| 10mM dNTPMix | 2µl |
| Total | 26µl |

The above solution was incubated at 65°C for 5 min to carry out hydridization between the dT30V oligomer and mRNA, followed by the addition of the following solution for the 1^{st} cDNA synthesis.

**[Table 9]**

| Solution for the 1^{st} cDNA synthesis | |
|---|---|
| 10X RT buffer | 5µl |
| 25mM MgCl₂ | 10µl |
| 0.1M DTT | 5µl |
| Rnase Out (40u/µl) | 2.5µl |
| SuperScript III RT (200u/µl) | 2.5µl |
| Total | 25µl |

After the reaction at 50°C for 60 min, 2µl of RNAse H(1u/µl) was added to the solution and heated at 50°C for 10 min, and then at 85°C for 5 min to inactivate the enzyme activity.

(h) Step of synthesizing a double-stranded cDNA with the single-stranded cDNA synthesized in the step (g) as a template wherein 5' end of a single-stranded cDNA complementarily synthesized is fixed to the solid phase:

Synthesized aRNA 2^{nd} oligomer modified with Biotin at its 5' (SEQ ID No.4)(2.8µg: about 250 pmol):
Biotin-GGGAATGGCTACACGAACTCGGTTCATGACACGG
and 100µl of Dynabeads M280 (Streptavidin-coated magnetic beads: DYNAL Co.) were mixed at a room temperature for 30 min to give rise to Biotin-Streptavidin reaction. After the resulting solution of the aRNA 2^{nd} oligomer fixed on the magnetic beads was placed and adsorbed onto magnet, supernatant was removed so as to exclude un-reacted aRNA 2^{nd} oligomer.

To the collected aRNA 2^{nd} oligomer fixed on the magnetic beads was added 27µl of the solution obtained in the step (g), and incubated at 65°C for 5 min to carry out hydridization between the aRNA 2^{nd} oligomer fixed on the magnetic beads and aRNA. After the resulting solution was placed and adsorbed onto the magnet, supernatant was removed so as to exclude unwanted enzyme and un-reacted agents. The magnetic beads were washed two times with 500µl of 0.1X TE buffer, and detached from the magnet. And the following solution for the 2^{nd} DNA synthesis was added thereto.

**[Table 10]**

| Solution for the 2^{nd} cDNA synthesis | |
|---|---|
| Water | 142µl |
| 5X 2^{nd} Strand buffer | 40µl |
| 10mM dNTPMix | 8µl |
| E. coli DNA polymerase (10u/µl) | 10µl |
| Total | 200µl |

The resulting solution was incubated at 16°C for 60 min, at 22°C for 60 min, and at 70°C for 10 min. To this was added 2µl of T4 DNA Polymerase (10u/µl) and incubated at 37°C for 10 min. The resulting solution was placed on magnet so that the magnetic beads would be adsorbed onto the magnet. Supernatant was then removed so as to exclude unwanted enzymes and un-reacted agents. The magnetic beads were washed two times with 500µl of 0.1X TE buffer. Finally, after the addition of water (176µl), the magnetic beads were detached from the magnet and dispersed in the water.

(i) Step of cleaving the double-stranded cDNA synthesized in the step (h) with a second restriction enzyme Y that does not cleave the X primer sequence at its 5' end:

**[Table 11]**

| Solution for cleavage with restriction enzyme MseI | |
|---|---|
| Solution of the step (h) | 176µl |
| 10X NEB2 buffer | 20µl |
| 100X BSA | 2µl |
| Mse I (10u/µl) | 2µl |
| Total | 200µl |

The above solution was reacted at 37°C for 4 hours.

(j) Step of purifying a double-stranded cDNA fragment fixed to the solid phase from the fragments prepared in the step (i) : After the solution prepared in the step (i) was placed and adsorbed onto the magnet, supernatant was removed so as to exclude unwanted enzyme and un-reacted agents. The magnetic beads were washed two times with 500µl of 0.1X TE buffer. Finally, after the addition of water (20µl), the magnetic beads were detached from the magnet and dispersed in the water.

(k) Step of preparing a double-stranded cDNA fragment having a Y adaptor bound to its 3' end by binding the X promoter-adaptor to a cleavage site with the second restriction enzyme Y in the double-stranded cDNA fragment purified in the step (j), wherein the Y adaptor comprises a sequence complementary to the cleavage site and a Y primer:

Preparation of Y adaptor
Synthesized DNA oligomer:
YS : TACGCAGTAGGACGCCTCGTGACGATACTT (SEQ ID No.5)
YSA : AAGTATCGTCACGAGGCGTCCTACTGCG (SEQ ID No.6)

50µl of each DNA oligomer (100pmol/µl) was mixed together, heated at 95°C for 5 min, and allowed to cool down to a room temperature so as to prepare solution of Y adaptor of the double-stranded DNA (50pmol/µl).

**[Table 12]**

| Ligation of Y adaptor | |
|---|---|
| Solution of the step (j) | 20µl |
| Y adaptor solution (50pmol/µl) | 2µl |
| 10X T4 DNA ligase buffer | 2.8µl |
| 10mM ATP | 1µl |
| T4 DNA ligase | 2µl |
| Total | 27.8µl |

The above solution was heated at 25°C for 4 hours and placed on the magnet so that the magnetic beads would be adsorbed onto the magnet. Supernatant was then removed so as to exclude unwanted enzymes and un-reacted agents. The magnetic beads were washed two times with 500µl of 0.1X TE buffer. Finally, after the addition of water (30µl), the magnetic beads were detached from the magnet and dispersed in the water.

(l) Step of amplifying the double-stranded cDNA fragment by 2⁷ to 2¹⁰ (about 128 - 1,024) times, which was prepared in the step (k) and comprising the sequence complementary to the X primer at its 5' end and the sequence complementary to the Y primer at its 3' end by means of PCR with a primer set of the X primer and Y primer:

Each solution comprising the following X primer and Y primer (100pmol/µl) was prepared, respectively.
X Primer (SEQ ID No.7)
TGGCTACACGAACTCGGTTC
Y Primer (SEQ ID No.8)
TCCTGCGGAGCACTGCTATG.

**[Table 13]**

| Solution for 2¹⁰-time amplification reaction | |
|---|---|
| Solution of the step (k) | 30µl |
| 10X PCR buffer | 5µl |
| 25mM MgCl₂ | 5µl |
| dNTP Mixture (2.5mM each) | 8µl |
| Taq Polymerase (5u/µl) | 1µl |
| X Primer (100pmol/µl) | 0.5µl |
| Y Primer (100pmol/µl) | 0.5µl |
| Total | 50µl |

The above reaction solution was set in an apparatus for PCR.

PCT Temperature steps:
Step 1: 95°C, 5 min;
Step 2: (95°C, 20 sec; 68°C, 15 min) x seven times;
Step 3: 60°C, 30 min.

The resulting solution was placed on the magnet so that the magnetic beads would be adsorbed onto the magnet. Supernatant comprising the amplified cDNA was then collected.

(m) Step of PCR with the use of the double-stranded cDNA sequence prepared in the step (1) as a template and a primer set of X1 primer comprising two-base sequence of N₁N₂ (N₁ and N₂ are a base selected from the group consisting of adenine, thymine, guanine and cytosine, being the same or different with each other) at its 3' end and Y1 primer comprising two base sequence of N₃N₄ (N₃ and N₄ are a base selected from the group consisting of adenine, thymine, guanine and cytosine, being the same or different with each other) at its 3' end:

X1 Primer: oligomer comprising a sequence complementary to the X adaptor and two-base sequence, and labled with fluorescent substance at its 5' end

**[Table 14]**

| | |
|---|---|
| FAM : | fluorescein label |
| HEX : | 5'-Hexafluorocerin label |
| NED: | fluorescent pigment label (Applied Biosystems Co.) |
| X-AA | FAM-ACTCGGTTCATGACACGG**AA** |
| X-AC | FAM-ACTCGGTTCATGACACGG**AC** |
| X-AG | FAM-ACTCGGTTCATGACACGG**AG** |
| X-AT | FAM-ACTCGGTTCATGACACGG**AT** |
| X-CA | NED-ACTCGGTTCATGACACGG**CA** |
| X-CC | NED-ACTCGGTTCATGACACGG**CC** |
| X-CG | NED-ACTCGGTTCATGACACGG**CG** |
| X-CT | HEX-ACTCGGTTCATGACACGG**CT** |
| X-GA | NED-ACTCGGTTCATGACACGG**GA** |
| X-GC | HEX-ACTCGGTTCATGACACGG**GC** |
| X-GG | HEX-ACTCGGTTCATGACACGG**GG** |
| X-GT | FAM-ACTCGGTTCATGACACGG**GT** |
| X-TA | NED-ACTCGGTTCATGACACGG**TA** |
| X-TC | HEX-ACTCGGTTCATGACACGG**TC** |
| X-TG | HEX-ACTCGGTTCATGACACGG**TG** |
| X-TT | FAM-ACTCGGTTCATGACACGG**TT** |

Y1 Primer: oligomer comprising a sequence complementary to the Y adaptor and two-base sequence

**[Table 15]**

| | |
|---|---|
| Y-AA | AGGCGTCCTACTGCGTAA**AA** |
| Y-AC | AGGCGTCCTACTGCGTAA**AC** |
| Y-AG | AGGCGTCCTACTGCGTAA**AG** |
| Y-AT | AGGCGTCCTACTGCGTAA**AT** |
| Y-CA | AGGCGTCCTACTGCGTAA**CA** |
| Y-CC | AGGCGTCCTACTGCGTAA**CC** |
| Y-CG | AGGCGTCCTACTGCGTAA**CG** |
| Y-CT | AGGCGTCCTACTGCGTAA**CT** |
| Y-GA | AGGCGTCCTACTGCGTAA**GA** |
| Y-GC | AGGCGTCCTACTGCGTAA**GC** |
| Y-GG | AGGCGTCCTACTGCGTAA**GG** |
| Y-GT | AGGCGTCCTACTGCGTAA**GT** |
| Y-TA | AGGCGTCCTACTGCGTAA**TA** |
| Y-TC | AGGCGTCCTACTGCGTAA**TC** |
| Y-TG | AGGCGTCCTACTGCGTAA**TG** |
| Y-TT | AGGCGTCCTACTGCGTAA**TT** |

Solution comprising each of the above 32 oligomers (2µM) were prepared. Each combination of X1 primer and Y1 primer (2µl each) was prepared in line accordance with the following table and divided into 256 PCR tubes, respectively.

**[Table 16]**

| Combination of X1 primers and Y1 primers | | | | | | | |
|---|---|---|---|---|---|---|---|
| X1 - Y1 | X1 - Y1 | X1 - Y1 | X1 - Y1 | X1 - Y1 | X1 - Y1 | X1 - Y1 | X1 - Y1 |
| AA - AA | AC - AA | AG - AA | AT - AA | TT - AA | GT - AA | CT - AA | TC - AA |
| AA - AC | AC - AC | AG - AC | - AC | TT - AC | GT - AC | CT - AC | TC - AC |
| AA - AG | AC - AG | AG - AG | - AG | TT - AG | GT - AG | CT - AG | TC - AG |
| AA - AT | AC - AT | AG - AT | AT - AT | TT - AT | GT - AT | CT - AT | TC - AT |
| AA - CA | AC - CA | AG - CA | AT - CA | TT - CA | GT - CA | CT - CA | TC - CA |
| AA - CC | AC - CC | AG - CC | AT - CC | TT - CC | GT - CC | CT - CG | TC - CC |
| AA - CG | AC - CG | AG - CG | AT - CG | TT - CG | GT - CG | CT - CG | TC - CG |
| AA - CT | AC - CT | AG - CT | AT - CT | TT - CT | GT - CT | CT - CT | TC - CT |
| AA - GA | AC - GA | AG - GA | AT - GA | TT - GA | GT - GA | CT - GA | TC - GA |
| AA - GC | AC - GC | AG - GC | AT - GC | TT - GC | GT - GC | CT - GC | TC - GC |
| AA - GG | AC - GG | AG - GG | AT - GG | TT - GG | GT - GG | CT - GG | TC - GG |
| AA - GT | AC - GT | AG - GT | AT - GT | TT - GT | GT - GT | CT - GT | TC - GT |
| AA - TA | AC - TA | AG - TA | AT - TA | TT - TA | GT - TA | CT - TA | TC - TA |
| AA - TC | AC - TC | AG - TC | AT - TC | TT - TC | GT - TC | CT - TC | TC - TC |
| AA - TG | AC - TG | AG - TG | AT - TG | TT - TG | GT - TG | CT - TG | TC - TG |
| AA - TT | AC - TT | AG - TT | AT - TT | TT - TT | GT - TT | CT - TT | TC - TT |
| TG - AA | CA - AA | GA - AA | TA - AA | CC - AA | CG - AA | GC - AA | GG - AA |
| TG - AC | CA - AC | GA - AC | TA - AC | CC - AC | CG - AC | GC - AC | GG - AC |
| TG - AG | CA - AG | GA - AG | TA - AG | CC - AG | CG - AG | GC - AG | GG - AG |
| TG - AT | CA - AT | GA - AT | TA - AT | CC - AT | CG - AT | GC - AT | GG - AT |
| TG - CA | CA - CA | GA - CA | TA - CA | CC - C | CG - CA | GC - CA | GG - CA |
| TG - CC | CA - CC | GA - CC | TA - CC | CC - CC | CG - CC | GC - CC | GG - CC |
| TG - CG | CA - CG | GA - CG | TA - CG | CC - CG | CG - CG | GC - CG | GG - CG |
| TG - CT | CA - CT | GA - CT | TA -CT | CC-CT | CG - CT | GC - CT | GG - CT |
| TG - GA | CA - GA | GA - GA | TA - GA | CC - GA | CG - GA | GC - GA | GG - GA |
| TG - GC | CA - GC | GA - GC | TA - GC | CC - GC | CG - GC | GC - GC | GG - GC |
| TG - GG | CA - GG | GA - GG | TA - GG | CC - GG | CG - GG | GC - GG | GG - GG |
| TG - GT | CA - GT | GA - GT | TA - GT | CC - GT | CG - GT | GC - GT | GG - GT |
| TG - TA | CA - TA | GA - TA | TA - TA | CC - TA | CG - TA | GC - TA | GG - TA |
| TG - TC | CA - TC | GA - TC | TA - TC | CC - TC | CG - TC | GC - TC | GG - TC |
| TG - TG | CA - TG | GA - TG | TA - TG | CC - TG | CG - TG | GC - TG | GG - TG |
| TG - TT | CA - TT | GA - TT | TA - TT | CC - TT | CG - TT | GC - TT | GG - TT |

**[Table 17]**

| PCR reaction solution | |
|---|---|
| Solution of the step (1) | 5µl |
| Water | 2,515µl |
| 1OX PCR buffer | 600µl |
| 25mM MgCl₂ | 600µl |
| dNTP Mixture (2.5mM each) | 960µl |
| Taq Polymerase (5u/µl) | 120µl |
| Total | 4,800µl |

PCR reaction solution (16µl) was divided into each tube and set in the apparatus for PCR.

PCT Temperature steps:
Step 1: 95°C, 1 min;
Step 2: (98°C, 20 sec; 71.5°C, 30 sec, 72°C, 1 min) x 28 times;
Step 3: 60°C, 30 min.

(n) Step of subjecting the PCR product to electrophoresis, and detecting migration length and peak so as to prepare the gene expression profile:
The PCR product prepared in the step (m) was subjected to electrophoresis and analysis using ABI PRISM (trade mark) 3100 Genetic Analyzer (Applied Biosystems Co.) in accordance with its manual. The analysis of the 256 tubes of each sample and each Lot revealed that electrophoresis patterns obtained in all of the samples coincided with each other for each combination of the X1 primer and Y1 primer. For example, Figures 12 ~ 14 show the results obtained in the case of X1 primer of X-AA and the Y1 primer of Y-GA using the samples T10uLot1 and T10uLot2, the samples T10nLot1 and T10nLot2 and the samples T20pLot1 and T20pLot2, respectively.

**[Table 18]**

| Sample Table | | | |
|---|---|---|---|
| Sample Name | Total RNA amount | Used amount (Concentration) | Number of cells (Conversion) |
| T10u | 10µg | 1 µl (10µg/µl) | 10⁶ |
| T10n | 10ng | 1 µl (10ng/µl) | 10³ |
| T20p | 20pg | 1 µl (20pg/µl) | 2 |

### Industrial applicability

The preparation according to the invention can be used in many fields, including gene analysis (SNPs analysis, DNA chip, PCR, etc.), a nano structure using nucleic acid, molecule machine, and nucleic acid medicine.

## Claims

1. A method for the preparation of gene expression profile comprising:
(a) a step of synthesizing a single-stranded cDNA whose 5' end is fixed to solid phase or which has a tag substance added to its 5' end with poly(A) RNA as a template;
(b) a step of synthesizing a double-stranded cDNA with the single-stranded cDNA synthesized in the step (a) as a template;
(c) a step of cleaving the double-stranded cDNA prepared in the step (b) with a first restriction enzyme X;
(d) a step of purifying a 3' end fragment that is fixed to the solid phase or has the tag substance added thereto from the double-stranded cDNA fragments prepared in the step (c);
(e) a step of preparing a double-stranded cDNA fragment having an X promoter-adaptor bound to its 5' end by binding the X promoter-adaptor to a cleavage site with the first restriction enzyme X in the fragment purified in the step (d), wherein the X promoter-adaptor comprises a sequence complementary to the cleavage site, an X primer sequence and a promoter sequence;
(f) a step of preparing an amplified RNA (aRNA) complementary to the double-stranded cDNA sequence prepared in the step (e) with the double-stranded cDNA fragment as a template by means of an RNA polymerase recognizing the promoter sequence;
(g) a step of synthesizing a single-stranded cDNA having a sequence complementary to the X primer with the aRNA synthesized in the step (f) as a template;
(h) a step of synthesizing a double-stranded cDNA whose 5' or 3' end is fixed to solid phase or which has a tag substance added to its 5' or 3' end with the single-stranded cDNA synthesized in the step (g) as a template;
(i) a step of cleaving the double-stranded cDNA synthesized in the step (h) with a second restriction enzyme Y that does not cleave the X primer sequence at its 5' end;
(j) a step of purifying a double-stranded cDNA fragment comprising a cleavage site with the second restriction enzyme Y at its 3' end from the fragments prepared in the step (i);
(k) a step of preparing a double-stranded cDNA fragment having a Y adaptor bound to its 3' end by binding the X promoter-adaptor to a cleavage site with the second restriction enzyme Y in the double-stranded cDNA fragment purified in the step (j), wherein the Y adaptor comprises a sequence complementary to the cleavage site and a Y primer;
(l) a step of amplifying the double-stranded cDNA fragment prepared in the step (k) by means of PCR with the use of said double-stranded cDNA fragment as a template and a primer set of the X primer and Y primer;
(m) a step of PCR with the use of the double-stranded cDNA sequence prepared in the step (1) as a template and a primer set of X1 primer comprising two-base sequence of N₁N₂ (N₁ and N₂ are a base selected from the group consisting of adenine, thymine, guanine and cytosine, being the same or different with each other) at its 3' end and Y1 primer comprising two base sequence of N₃N₄ (N₃ and N₄ are a base selected from the group consisting of adenine, thymine, guanine and cytosine, being the same or different with each other) at its 3' end; and
(n) a step of subjecting the PCR product prepared in the step (m) to electrophoresis, and detecting migration length and peak so as to prepare the gene expression profile.

2. A method for the preparation of gene expression profile comprising:
(a) a step of synthesizing a single-stranded cDNA whose 5' end is fixed to solid phase or which has a tag substance added to its 5' end with poly(A) RNA as a template;
(b) a step of synthesizing a double-stranded cDNA with the single-stranded cDNA synthesized in the,step (a) as a template;
(c) a step of cleaving the double-stranded cDNA prepared in the step (b) with a first restriction enzyme X;
(d) a step of purifying a 3' end fragment that is fixed to the solid phase or has the tag substance thereto from the double-stranded cDNA fragments prepared in the step (c) ;
(e) a step of preparing a double-stranded cDNA fragment having an X promoter-adaptor bound to its 5' end by binding the X promoter-adaptor to a cleavage site with the first restriction enzyme X in the fragment purified in the step (d), wherein the X promoter-adaptor comprises a sequence complementary to the cleavage site, an X primer sequence and a promoter sequence;
(f) a step of preparing an amplified RNA (aRNA) complementary to the double-stranded cDNA sequence prepared in the step (e) with the double-stranded cDNA fragment as a template by means of an RNA polymerase recognizing the promoter sequence;
(g) a step of synthesizing a single-stranded cDNA having a sequence complementary to the X primer with the aRNA synthesized in the step (f) as a template;
(h) a step of synthesizing a double-stranded cDNA whose 5' or 3' end is fixed to solid phase or which has a tag substance added to its 5' or 3' end with the single-stranded cDNA synthesized in the step (g) as a template;
(i) a step of cleaving the double-stranded cDNA synthesized in the step (h) with a second restriction enzyme Y that does not cleave the X primer sequence at its 5' end;
(j) A step of purifying a double-stranded cDNA fragment comprising a cleavage site with the second restriction enzyme Y at its 3' end from the fragments prepared in the step (i);
(k) a step of preparing a double-stranded cDNA fragment having a Y adaptor bound to its 3' end by binding the X promoter-adaptor to a cleavage site with the second restriction enzyme Y in the double-stranded cDNA fragment purified in the step (j), wherein the Y adaptor comprises a sequence complementary to the cleavage site and a Y primer;
(m) a step of PCR with the use of the double-stranded cDNA sequence prepared in the step (1) as a template and a primer set of X1 primer comprising two-base sequence of N₁N₂ (N₁ and N₂ are a base selected from the group consisting of adenine, thymine, guanine and cytosine, being the same or different with each other) at its 3' end and Y1 primer comprising two base sequence of N₃N₄ (N₃ and N₄ are a base selected from the group consisting of adenine, thymine, guanine and cytosine, being the same or different with each other) at its 3' end; and
(n) a step of subjecting the PCR product prepared in the step (m) to electrophoresis, and detecting migration length and peak so as to prepare the gene expression profile.

3. A method according to Claims 1 or 2, wherein the double-stranded cDNA whose 5' end is fixed to solid phase or which has the tag substance added to its 5' end is synthesized in the step (h) with the single-stranded cDNA synthesized in the step (g) as a template, by using an oligomer comprising the X primer sequence or a part thereof fixed to the solid phase or having the tag substance added thereto as a primer for a complementarily synthesized cDNA.

4. A method according to Claims 1 or 2, wherein the double-stranded cDNA whose 3' end is fixed to solid phase or which has tag substance added to its 3' end is synthesized in the step (h) with the single-stranded cDNA synthesized in the step (g) as a template, by using an oligo T primer fixed to the solid phase or having the tag substance added thereto in the step (g).

5. A method according to any one of Claims 1 to 4, wherein the promoter sequence is T7 promoter sequence.

6. A method for the preparation of gene expression profile comprising:
(a) a step of synthesizing a single-stranded cDNA whose 5' end is fixed to solid phase or which has a tag substance added to its 5' end with poly(A) RNA as a template;
(b) a step of synthesizing a double-stranded cDNA with the single-stranded cDNA synthesized in the step (a) as a template;
(c) a step of cleaving the double-stranded cDNA prepared in the step (b) with a first restriction enzyme X;
(d) a step of purifying a 3' end fragment that is fixed to the solid phase or has the tag substance added thereto from the double-stranded cDNA fragments prepared in the step (c);
(e) a step of preparing a double-stranded cDNA fragment having an X promoter-adaptor bound to its 5' end by binding the X promoter-adaptor to a cleavage site with the first restriction enzyme X in the fragment purified in the step (d), wherein the X promoter-adaptor comprises a sequence complementary to the cleavage site, an X primer sequence and a promoter sequence;
(i) a step of cleaving the double-stranded cDNA synthesized in the step (e) with a second restriction enzyme Y that does not cleave the X primer sequence at its 5' end;
(j) A step of purifying a double-stranded cDNA fragment comprising a cleavage site with the second restriction enzyme Y at its 3' end from the fragments prepared in the step (i);
(k) a step of preparing a double-stranded cDNA fragment having a Y adaptor bound to its 3' end by binding the X promoter-adaptor to a cleavage site with the second restriction enzyme Y in the double-stranded cDNA fragment purified in the step (j), wherein the Y adaptor comprises a sequence complementary to the cleavage site and a Y primer;
(l) a step of amplifying the double-stranded cDNA fragment prepared in the step (k) by means of PCR with the use of said double-stranded cDNA fragment as a template and a primer set of the X primer and Y primer;
(m) a step of PCR with the use of the double-stranded cDNA sequence prepared in the step (1) as a template and a primer set of X1 primer comprising two-base sequence of N₁N₂ (N₁ and N₂ are a base selected from the group consisting of adenine, thymine, guanine and cytosine, being the same or different with each other) at its 3' end and Y1 primer comprising two base sequence of N₃N₄ (N₃ and N₄ are a base selected from the group consisting of adenine, thymine, guanine and cytosine, being the same or different with each other) at its 3' end; and
(n) a step of subjecting the PCR product prepared in the step (m) to electrophoresis, and detecting migration length and peak so as to prepare the gene expression profile.

7. A method according to any one of Claims 1 to 5, wherein in the step (f) the amplified RNA is prepared in an amount of about 10 to 500 times as much as the number of the double-stranded cDNA fragments.

8. A method according to any one of Claims 1 or 3 to 6, wherein in the step (1) PCR is repeated in 7 to 10 cycles so that the number of the double-stranded cDNA fragment shall be amplified by 128 to 1,024 times.

9. A method according to any one of Claims 1 to 8, which uses each DNA sequence fixed to the solid phase.

10. A method according to any one of Claims 1 to 8, which uses each DNA sequence having the tag substance added thereto.
